# EUROPEAN PATENT APPLICATION

(11) **EP 2 944 352 A1**
(43) Date of publication of application: **18.11.2015**
(21) Application number: 15166506.4
(22) Date of filing: 06.05.2015
(51) Int. Cl.: A61N 1/372, A61N 1/375

(54) **INNER PACKAGING FOR A STERILIZABLE CONTAINER AND STERILIZABLE CONTAINER**

(30) Priority: 14.05.2014 US 201461992930 P; 09.01.2015 US 201562101378 P
(71) Applicant: BIOTRONIK SE & Co. KG, 12359 Berlin (DE)
(72) Inventor: Moulder, J. Christopher, Portland, OR Oregon 97202 (US); Kibler, Andrew, Lake Oswego, OR Oregon 97035 (US)
(74) Representative: Galander, Marcus

(57) **Abstract**

The invention concerns a sterilizable container for an implantable medical device. The container comprises an inner packaging and an outer packaging that encloses said inner packaging. The inner packaging comprises an enclosure and at least two electric contacts that are arranged inside the enclosure and that are placed so as to contact an implantable medical device electrode when such implantable medical device is placed in said inner packaging. Each electric contact of said inner packaging is electrically connected to a respective planar electrode arranged at or close to an outer surface of said inner packaging so as to provide a capacitive communication interface. The inner packaging further comprises fixture means that are configured to securely hold an implantable medical device in place to ensure electric contact between the at least two electric contacts and a respective implantable medical device electrode when such implantable medical device is placed in the inner packaging.

## Description

The invention refers to a sterilizable container for an implantable medical device. The invention further refers to a sterilizable container containing an implantable medical device. In particular, the invention refers to a sterilizable container for an implantable programmable heart stimulator, such as an implantable pacemaker or an implantable cardioverter/defibrillator. The invention further refers to a method of programming and/or testing an implantable medical device.

Implantable medical devices must be sterilized prior to use. Therefore, a container is needed, wherein the implantable medical device can be maintained sterile. To ensure that the implantable medical device is maintained in a sterile condition, the implantable medical device is sterilized together with a container enclosing the device.

In some examples, implantable medical devices are implantable leadless pacemakers (iLP) that are small enough to be placed within a ventricle of a human heart. In some examples, implantable leadless pacemakers feature two electrode poles that are arranged on the pacemaker's casing and that are configured to deliver stimulation pulses to a conductive medium or tissue surrounding the implantable leadless pacemaker when in use. In contrast to iLP's, traditional implantable pacemakers utilize electrode leads which contain electrodes at or near their distal end. These electrode leads are connected to the implantable pacemaker and can be implanted and explanted separately from the pacemaker. Implantable leadless pacemakers, in contrast, utilize no electrode leads and therefore only one component needs to be implanted and is required to be kept sterile prior to implantation.

It is an object of the invention to provide a suitable container for an implantable medical device, and in particular for a programmable implantable leadless pacemaker.

According to the invention, this object is achieved by means of an inner packaging for a sterilizable container for an implantable medical device. The inner packaging comprises an enclosure and at least two electric contacts that are arranged inside the enclosure and that are placed so as to contact an implantable medical device electrode when such implantable medical device is placed in said inner packaging. Each electric contact of said inner packaging is electrically connected to a respective planar electrode arranged at or close to an outer surface of said inner packaging so as to provide a capacitive communication interface. The inner packaging further comprises fixture means that are configured to securely hold an implantable medical device in place to ensure electric contact between the at least two electric contacts and a respective implantable medical device electrode when such implantable medical device is placed in the inner packaging.

The object is further achieved by sterilizable container for an implantable medical device. The container comprises an inner packaging as disclosed above and an outer packaging that encloses said inner packaging.

The object of the invention is still further achieved by a method that comprises the steps:
- providing an implantable medical device within a sterilized container as pointed out above,
- placing data communication electrodes in the proximity of the planar electrodes of the inner packaging
- programming and/or testing the implantable medical device within the closed container via capacitive coupling between the communication electrodes and the planar electrodes of the inner packaging.

The invention meets the desire to communicate with an implantable medical device before implant while it is still in the sterile packaging in order to perform pre-implant tests and programming. Methods of communication applied in the art of implantable pacemaker devices utilize RF or magnetic communication, both of which readily penetrate sterile packaging layers to perform this pre-implant communication function. Recent device miniaturization advances and applications have required reductions in communications power and increases in device communication depth within the body. These changes have required the invention of novel forms of communication via electrical fields which do not readily penetrate sterile packaging.

The invention provides a communication system and method which facilitates the transmission of electric field based methods of communication from the electrodes on a device across sterile packaging, potentially multiple layers of standard, unmodified sterile packaging, to a communications unit outside of the sterile packaging.

Known solutions for device-communication include capacitive coupling across a sterile barrier and electrical contact through a sterile barrier as taught in US 8,219,200, and conductive liquid coupling (glycerol) as taught in US 6,116,413, inductive coupling as taught in US 2012/0112690, wires which penetrate the sterile packaging and provide electrical connection to the implantable device as taught in US 8,224,447 and wireless antenna coupling across sterile packaging as taught in US 7,289,855. The inventors have recognized that the solutions known so far typically require a modification of standard sterile containers.

The invention provides in particular a packaging and a container that allow communication with implantable medical devices that provide for an electric field based data communication, in particular those micro-sized implantable medical devices that rely on electrical field based communication which does not easily penetrate sterile packaging. Testing of and communicating with an implantable medical device while it is still in a sterile package, without significant modifications to the sterile package or multiple payers of the sterile package is achieved by means of the inventive inner packaging and the container comprising such inner packaging which maintains sterility and allows communication to the device as required.

The inventors have further recognized that wireless antenna coupling across a sterile packaging as described in US 7,289,855 is applicable when communicating at RF frequencies and with sufficient power, however these frequencies are not optimal for intra-body propagation. Communications carrier frequencies which are optimal for intra-body propagation poorly couple electromagnetically between antennas in air. Therefore communication across the sterile packaging would require increased power or greatly increased antenna size to facilitate electromagnetic coupling. The invention provides a solution that allows data communication with carrier frequencies which are optimal for intra-body propagation without requiring increased power.

Preferred embodiments of the invention include an inner packaging, wherein at least one of the electric contacts is a soft conductive interface plug that is configured to contact a first electrode of the implantable device to make electrical contact without damaging the first electrode of the implantable device or an electrode coating. Thus it is achieved that the electrical contact to the implantable device electrode does not damage sensitive coatings or put significant pressure on sensitive flexible components such as screw electrode. Common compression interfaces are not adequate to preserve the screw electrode.

Preferably, the soft conductive interface plug comprises rubber or a gel material or both. The rubber is preferably silicone-based. The gel material preferably comprises glycerol.

According to further preferred embodiments of the inner packaging the fixture means for securely holding an implantable medical device in place comprise a rigid frame arranged inside the inner packaging. At least one of the electric contacts is mounted in the rigid frame to ensure reliable electric contact between each respective electric contact and a respective implantable medical device electrode through sterilization and transportation.

Preferably, the rigid frame is made from plastic material.

According to further preferred embodiments of the inner packaging each planar electrode has an electrode surface of more than 10 cm² or more than 20 cm² and less than 50 cm². A preferred size of the planar electrodes is between 20 cm² and 35 cm².

Preferably, the inner packaging contains an implantable medical device. The implantable medical device is preferably configured for impedance-based data communication with an external device. In a further preferred embodiment the implantable medical device is an implantable leadless pacemaker having at least one screw or spring electrode that contacts the soft conductive interface plug.

The invention provides an interface across the sterile container comprising an inner packaging and an external communication wand which allows the medium frequency communications pulses to be capacitively coupled, allowing bidirectional communication across the sterile membrane without requiring modifications to the sterile container. The inner packaging contains at least two planar electrodes which serve to enlarge the electrical surface area available for coupling across the sterile container. In addition, a soft conductive interface plug is inserted in contact with a first electrode of the implantable device (screw of iLP) to make electrical contact without damaging the electrode or electrode coating. The plug may be composed of rubber (possibly silicone-based) or a gel (glycerol) material. A second contact is made with a second electrode of the device (possibly the fin of an iLP) and both contacts are mounted in a rigid frame of the inner packaging for reliable contact through sterilization and transportation.

Communication is facilitated between the inner packaging, inside the sterile container, and an external communications wand adapted to couple to the inner fixture across the sterilization package. The external wand comprises two communication electrodes that can couple capacitively to the planar electrodes of the inner packaging. The external wand is not a component of the container, but is applied across the walls of the container such that is at least two communication electrodes are positioned in alignment with the at least two planar electrodes on the other side of the container wall. Coupling, potentially magnetic, between the inner packaging and the external wand facilitates this alignment and compresses the sterile container such that the reduced inter-electrode distance allows capacitive coupling between the wand communication electrodes and the planar electrodes of the inner packaging, facilitating communication.

Preferably, the spacing between the wand electrodes and the planar electrodes of the inner packaging is less than 5 mm, preferably 1mm to 5mm, to facilitate capacitive coupling.

According to another embodiment of the invention, coupling and alignment between the inner packaging and the wand is facilitated by 3-dimensional features patterned into the inner or outer packaging and wand such that the features nest together when proper alignment is achieved.

According to a preferred embodiment of the method, the method further comprises the steps of:
- placing the implantable medical device into a container,
- closing the container containing the implantable medical device,
- sterilizing the container containing the implantable medical device.

Thus an implantable medical device within a sterilized container is provided that can be tested or programmed by means of the method steps mentioned supra.

The above and other aspects, features and advantages of the present invention will be more apparent from the following more particular description thereof, presented in conjunction with the following figures. Of the Figures,
- Fig. 1: illustrates an inner packaging according the invention, containing an implantable medical device; and
- Fig. 2: illustrates a sterile container according to the invention, containing an inner packaging as illustrated in Fig. 1.
- Fig. 3: illustrates a sterile container according to the invention, containing an inner packaging as illustrated in Fig. 1, with magnetic coupling between the wand and packaging.
- Fig. 4: illustrates a sterile container according to the invention, containing an inner packaging as illustrated in Fig. 1, with 3-dimensional features facilitating coupling alignment between the wand and packaging.

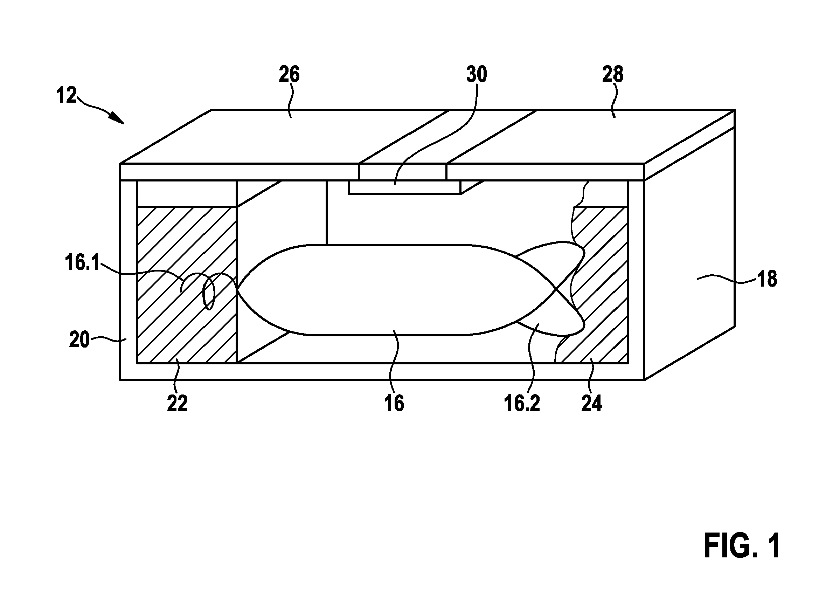

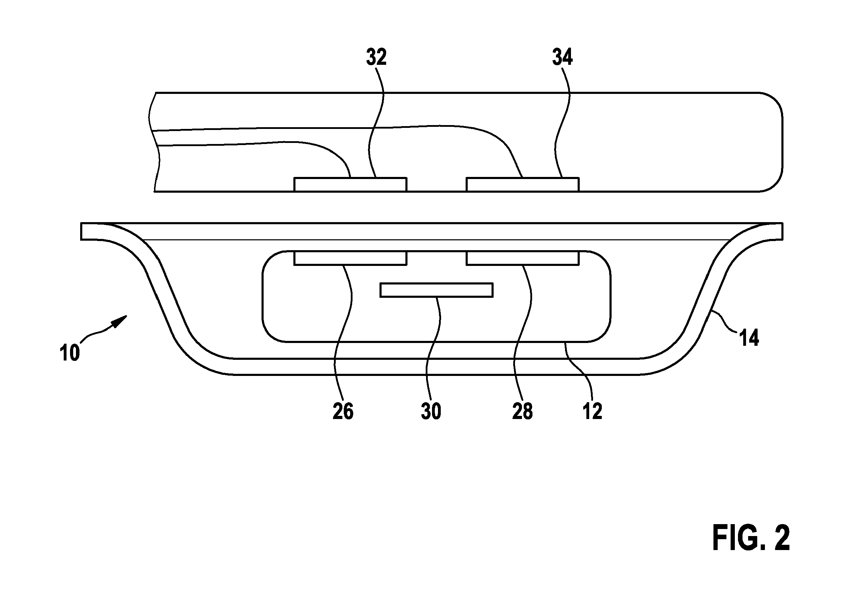

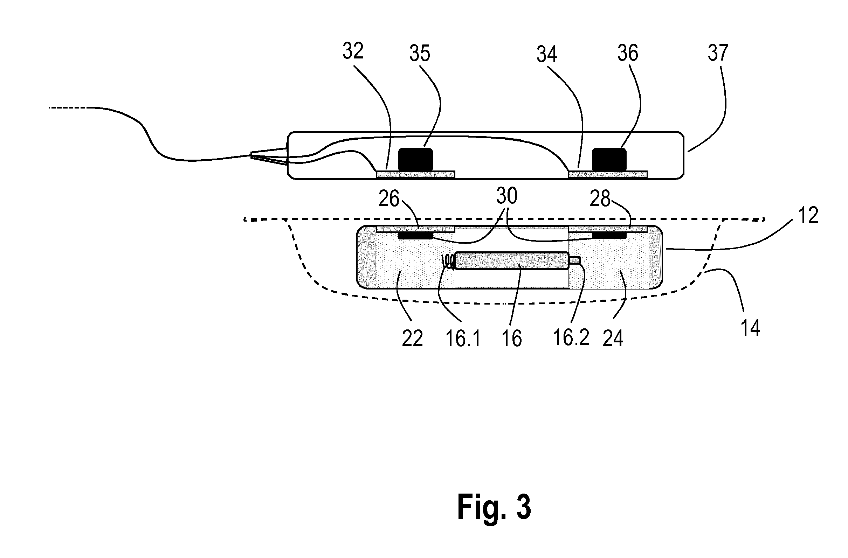

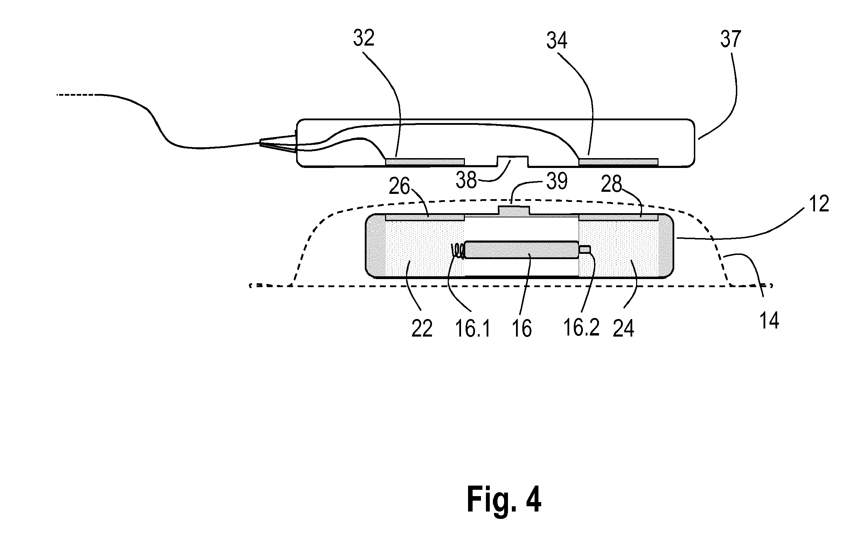

Figs. 1, 2, 3, and 4 depict a sterile container 10 (Fig. 2) that comprises an inner packaging 12 and an outer packaging 14 that encloses the inner packaging 12.

Fig. 1 illustrates the details of inner packaging 12. Inner packaging 12 contains an implantable medical device 16 within an enclosure 18. It is noted that the enclosure 18 and thus the inner packaging 12 can be fully closed or not. In particular, it is possible that the enclosure is a mere frame or other fixture means for holding the implantable medical device 16.

In the exemplary embodiment illustrated by Figs. 1 and 2, the implantable medical device 16 is an implantable leadless pacemaker as an exemplary implantable device in the sterile container 10. The implantable leadless pacemaker 16 has a first (implantable medical device) electrode 16.1 for contacting cardiac tissue, such as a screw, spring, or flat electrode that is sensitive to mechanical stress and a second (implantable medical device) electrode 16.2 that is a fin electrode and that can sustain more mechanical stress than the first electrode 16.1.

The implantable leadless pacemaker 16 is mounted in the inner packaging 12. The inner packaging 12 comprises fixture means that comprise a plastic frame 20. The plastic frame 20 can be integral part of enclosure 18 of the inner packaging 12.

The plastic frame 20 bears two electric contacts 22 and 24, one for each of the implantable medical device electrodes 16.1 and 16.2. The first electric contact 22 is placed in electrical communication with the implantable medical device's spring or screw electrode 16.1. This first electric contact 22 is composed of a soft material so as to not damage the spring coating or the electrode surface of the implantable medical device electrode 16.1. The first electric contact 22 thus is a soft conductive interface plug made from soft material, that is a biocompatible, stable, non-evaporative gel or a conductive, biocompatible silicon.

Plastic frame 20 further houses the second electric contact 24 in electrical communication with a fin electrode of the implantable leadless pacemaker, said fin electrode being the second implantable medical device electrode 16.2. The second electric contact 24 is configured to make electrical contact to the second implantable medical device electrode 16.2 via compression against the second medical implant electrode 16.2.

The two electric contacts 22 and 24 are arranged on the plastic frame 20 so as to provide positive electric contact to the implantable medical device electrodes 16.1 or 16.2, respectively, while the implantable medical device 16 is securely held by the plastic frame.

The two electric contacts 22 and 24 are electrically connected to two planar electrodes 26 and 28. The electric connection between each electric contact 22 or 24, respectively, to a respective planar electrode 26 or 28 can be provided by a wire or any other electrically conducting material.

The planar electrodes 26 and 28 are arranged inside the inner packaging 10 at or close to an enclosure wall of enclosure 18. Each planar electrode 26 or 28 has a surface of approximately 30 cm². The spacing between the two planar electrodes is approximately 2 cm.

The planar electrodes 26 and 28 serve as trans-packaging communication electrodes.

Preferably, the inner packaging 12 further houses at least one ferromagnetic slug 30, which facilitates alignment of the inner packaging 12 and the planar electrodes 26 and 28 thereof, with an alignment magnet in an external wand.

The external wand (not shown) comprises two communication electrodes 32 and 34 (see Fig. 2) that can capacitively couple to the planar electrodes 26 and 28 to allow wireless data communication.

A trans-packaging communication system thus comprises a communications wand (not shown) with two communication electrodes 22 and 24 that are sized and spaced to align with the two planar electrodes 22 and 24 of the inner packaging.

Preferable, the wand (not shown) preferably further comprises a magnet (not shown), which facilitates alignment of the wand communication electrodes 32 and 34 with the planar electrodes 22 and 24 of the inner packaging by means of the ferromagnetic slug 30 of the inner packaging. Thus, reduced inter-electrode distance between the wand communication electrodes 32 and 34 and the planar electrodes 22 and 24 of the inner packaging 12 can be achieved by means of compressing the container 10.

In such arrangement, capacitance in the order of nF develops across the sterile container 10 between each communication electrode 32 or 34, respectively, and a respective planar electrode 22 or 24 of the inner packaging 12, facilitating electrical communication to and from the implantable medical device 16.

According to the preferred embodiment, electrical communication takes place at below 1 MHz, preferably between 216 kHz and 400 kHz, or at or about 400 kHz, which sufficiently crosses the capacitance of the container interface to allow data communication.

This method of trans-package communication is compatible with both impedance-based communication and continuous wave-based communication.

The container according to the invention enables wireless electric data communication and implantable medical device programming while it is still in its sterile container. This technology allows to bring miniature implantable devices to market, which use low-power wireless electric communication (both impedance type and continuous wave type). The container according to the invention does not require modifications to the outer packaging to enable communication.

### Reference List

- 10: sterile container
- 12: inner packaging
- 14: outer packaging
- 16: implantable medical device
- 16.1: first implantable medical device electrode (spring, screw)
- 16.2: second implantable medical device electrode (fin)
- 18: enclosure
- 20: plastic frame
- 22, 24: electric contact
- 26, 28: planar electrode
- 30: ferromagnetic slug
- 32, 34: wand communication electrode
- 35, 36: alignment magnet
- 37: communication wand
- 38: wand alignment feature
- 39: inner packaging alignment feature

## Claims

1. Inner packaging (12) for a sterilizable container (10) for an implantable medical device (16), said inner packaging (12) comprising an enclosure (18) and at least two electric contacts (22, 24) that are arranged inside the enclosure (18) and placed so as to contact an implantable medical device electrode (16.1, 16.2) when such implantable medical device (16) is placed in said inner packaging (12), each electric contact (22, 24) of said inner packaging (12) being electrically connected to a respective planar electrode (26, 28) arranged at or close to an enclosure wall of said enclosure (18) so as to provide a capacitive communication interface; said inner packaging (12) further comprising fixture means that are configured to securely hold an implantable medical device (16) in place to ensure electric contact between said at least two electric contacts (22, 24) and a respective implantable medical device electrode (16.1, 16.2) when such implantable medical device (16) is placed in said inner packaging (12).

2. Inner packaging (12) according to claim 1, wherein at least one of the at least two electric contacts (22, 24) is a soft conductive interface plug (22) that is configured to contact a first electrode (16.1) of the implantable device (16) to make electrical contact without damaging the first electrode (16.1) of the implantable device (16) or an electrode coating.

3. Inner packaging (12) according to claim 2, wherein the soft conductive interface plug (22) comprises rubber or a gel material or both.

4. Inner packaging (12) according to claim 3, wherein the rubber is silicone-based and wherein the gel material comprises glycerol.

5. Inner packaging (12) according to claim 1, wherein the fixture means for securely holding an implantable medical device (16) in place comprise a rigid frame (20) and wherein at least one of the at least two electric contacts (22, 24) or both of the at least two electric contacts (22, 24) are mounted in said rigid frame (20) to ensure reliable electric contact between each respective electric contact and a respective implantable medical device electrode (161, 16.2) through sterilization and transportation.

6. Inner packaging (12) according to claim 5, wherein said rigid frame (20) is made from plastic material.

7. Inner packaging (12) according to claim 1, wherein each planar electrode (26, 28) has an electrode surface of more than 10 cm² or more than 20 cm² and less than 50 cm².

8. Inner packaging (12) according to claim 1, containing an implantable medical device (16), wherein the implantable medical device (16) is configured for impedance-based data communication with an external device.

9. Inner packaging (12) according to claim 2 and claim 8, wherein said implantable medical device (16) is an implantable leadless pacemaker having at least one screw or spring electrode that contacts said soft conductive interface plug (22).

10. System of communicating to an implantable medical device contained in a sterile package according to claim 1 via a wand comprising two communication contacts wherein the contacts are adapted to be aligned between the sterile packaging and the wand.

11. System of claim 10 further comprising magnetic couplers to align the contacts.

12. System of claim 10 wherein the package comprises 3D feature nesting and/or visual alignment markers.

13. Sterilizable container (10) for an implantable medical device (16), said container (10) comprising an inner packaging (12) according to at least one of claims 1 to 9 and an outer packaging (14) that encloses said inner packaging (12).

14. A method of programming and/or testing an implantable medical device, said method comprising:
- providing an implantable medical device within a sterilized container according to claim 13,
- placing data communication electrodes in the proximity of the planar electrodes of the inner packaging
- programming and/or testing the implantable medical device within the closed container via capacitive coupling between the communication electrodes and the planar electrodes of the inner packaging.

15. The method of claim 14, further comprising the steps of
- placing the implantable medical device into a container according to claim 13,
- closing the container containing the implantable medical device
- sterilizing the container containing the implantable medical device.
